# EUROPEAN PATENT SPECIFICATION

(11) **EP 2 260 891 B1**
(45) Date of publication and mention of the grant of the patent: **28.03.2018**
(21) Application number: 09719433.6
(22) Date of filing: 04.02.2009
(51) Int. Cl.: A61M 5/00, A61M 5/145

(54) **SYRINGE PUMP**
SPRITZENPUMPE
POMPE À SERINGUE

(30) Priority: 14.03.2008 JP 2008066690; 14.03.2008 JP 2008066689
(43) Date of publication of application: 15.12.2010
(60) Divisional application: 13179610.4
(73) Proprietor: Terumo Kabushiki Kaisha, Tokyo 151-0072 (JP)
(72) Inventor: HASEGAWA, Eiji, Fujinomiya-shi Shizuoka 418-0015 (JP)
(74) Representative: Gill, Stephen Charles
(86) International application number: PCT/JP2009/051856
(87) International publication number: WO 2009/113341

(56) References cited:
- JP-A- 6 190 036
- JP-A- 2001 245 974
- JP-A- 2002 113 099
- US-A1- 2003 045 789

## Description

### TECHNICAL FIELD

The present invention relates to a syringe pump and a method of controlling the same.

### BACKGROUND ART

A syringe pump is used in intensive care units (ICUs) and the like and has as its main object to perform nutritional support for a patient, blood infusion, infusion of a medicinal solution such as a chemotherapeutic agent or anesthetic medicinal solution with high accuracy for a relatively long period of time. Medicinal solution flow rate control or the like for this syringe pump is superior to pumps of other infusion forms, and hence is frequently used. A method of using a syringe pump includes setting the syringe main body of a syringe filled with a medicinal solution in a stationary state by using a clamp for the syringe pump, setting a syringe plunger in a slider assembly, and accurately feeding the content of the syringe, for example, a medicinal solution, accompanying the piston motion of the syringe plunger by pressing/driving the syringe plunger after manually moving the slider assembly to an infusion start position.

There has been proposed a syringe pump configured to detect that the syringe plunger is set on the slider assembly of the syringe pump and manually move the slider assembly to an infusion start position as an initial position (PLT1). In addition, there has been proposed a syringe pump configured to detect that the syringe main body is properly set on the mount base of the syringe pump (PLT2). Furthermore, since an occluded state occurs in an infusion system including an infusion line during infusion, the present applicant has proposed a syringe pump configured to automatically change an occlusion detection level in accordance with the position of the syringe plunger by detecting the occluded state (PLT3).

### CITATION LIST

### PATENT LITERATURE

PLT1: PCT(WO) 9-506288
PLT2: Japanese Patent Laid-Open No. 2004-73373
PLT3: PCT(WO) 0:2/ 066102 A1, which discloses the features of the preamble of claim 1.

US 2003/0045789 proposes syringe interfaces for variable size syringes and a drive including such interfaces.

### SUMMARY OF INVENTION

### TECHNICAL PROBLEM

The syringe pump disclosed in PLT3 can maintain an optimal occlusion detection level after the occurrence of an occluded state in an infusion system including an infusion line during infusion. When, however, the internal syringe pressure further increases, it is necessary to reduce the internal syringe pressure by driving the syringe plunger in a direction opposite to that of infusion.

This reduction in internal syringe pressure is required when, for example, a medicinal solution is administered into a patient through a three-way valve during, for example, artificial dialysis. If the internal syringe pressure is reduced in the above manner when the remaining amount of medicinal solution in the syringe volume is small, reducing the internal syringe pressure in the above manner will draw blood from the patient at worst.

### SOLUTION TO PROBLEM

The present invention has therefore been made in consideration of the above situations, and has as its object to provide a syringe pump with a superior level of safety by controlling the reducing operation of driving the slider assembly in a direction opposite to that of infusion to reduce the internal syringe pressure, and a method of controlling the syringe pump.

In order to solve the above problem and achieve the above object, according to the present invention, there is provided a syringe pump according to claim 1.

The syringe pump is characterized in that an amount of driving in the opposite direction is determined based on a magnitude of the syringe volume by the syringe volume detection means.

### ADVANTAGEOUS EFFECTS OF INVENTION

According to the present invention, it is possible to ensure safety by stopping the reducing operation of the internal pressure reduction means when the accumulated quantity is reduced and becomes less than or equal to a specified value in response to operation of the internal pressure reduction means.

Other features and advantages of the present invention will be apparent from the following description taken in conjunction with the accompanying drawings, in which like reference characters designate the same or similar parts throughout the figures thereof.

### BRIEF DESCRIPTION OF DRAWINGS

The accompanying drawings, which are incorporated in and constitute a part of the specification, illustrate embodiments of the invention and, together with the description, serve to explain the principles of the invention.

Fig. 1A is a plan view of a syringe type infusion device 1 (to be also referred to as the device 1 hereinafter);
Fig. 1B is a front view of the device 1;
Fig. 2 is a plan view of an operation panel 10 of the device 1;
Fig. 3A is a plan view of the device 1;
Fig. 3B is a rear view of the device 1;
Fig. 3C is a right side view of the device 1;
Fig. 4 is a perspective view showing an outer appearance of how the devices 1 are fixed to mounting tools 300 fixed to an infusion stand 200;
Fig. 5 is a perspective view showing the outer appearance of a state after the devices 1 are fixed to the mounting tools 300 fixed to the infusion stand 200, together with syringes S;
Fig. 6 is a schematic view of a slider feeding mechanism 70 built in the device 1;
Fig. 7 is a block diagram of the syringe type infusion device 1;
Fig. 8 is a perspective view showing the outer appearance of how a rechargeable battery 40 of a lithium-ion battery as a secondary battery is stored in the battery storage portion formed in the bottom surface of a lower cover 3;
Fig. 9 is a flowchart for explaining operation including the execution of refresh of the battery;
Fig. 10 is a flowchart showing a case in which a serviceman or the like manually executes refresh of the battery;
Fig. 11A is a view showing an example of a display window on a display unit 20 at the time of the execution of refresh of the battery described above;
Fig. 11B is a view showing an example of a display window indicating the voltage trend of the battery after the execution of refresh for a period of 72 months up to the present;
Fig. 11C is a view showing an example of a display window showing how a liquid crystal backlight blinks to prompt replacement;
Fig. 12 is a flowchart for explaining the operation of an occlusion management means, showing an example of the operation of making a display unit 20 display internal syringe pressures at least at 1-min intervals in chronological order, together with an occlusion degree;
Fig. 13 is a flowchart for explaining the operation of determining that there is a possibility that occlusion abnormality may occur in an infusion line to which a syringe is connected, by detecting an internal syringe pressure at the time of infusion;
Fig. 14A is a view showing an example of a display window on the display unit 20 which displays occlusions from the start time of infusion to the present, together with a set occlusion degree;
Fig. 14B is a view showing an example of display window on the display unit 20 which displays a warning against the possibility of the occurrence of an occlusion; and
Fig. 15 is a flowchart for explaining the operation of an occlusion reduction means 116.

### BEST MODE FOR CARRYING OUT THE INVENTION

An embodiment of the present invention will be described below with reference to the accompanying drawings.

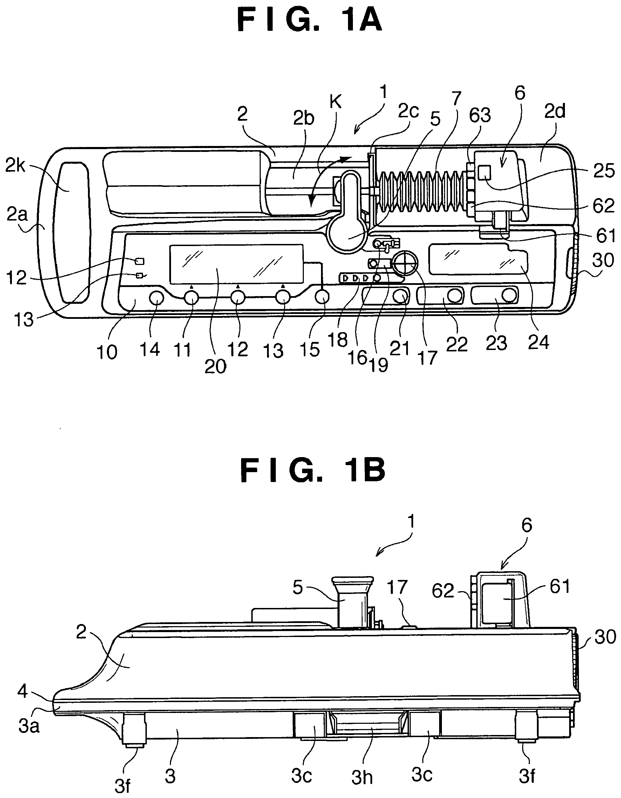

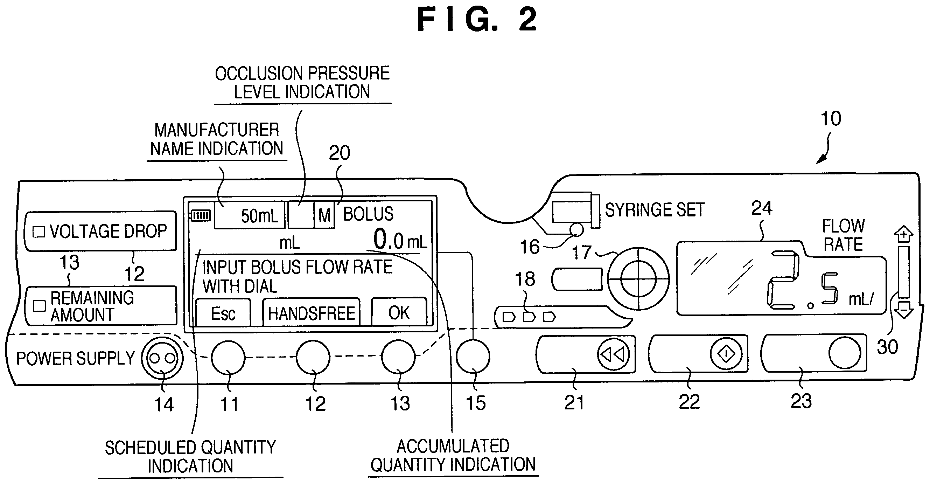

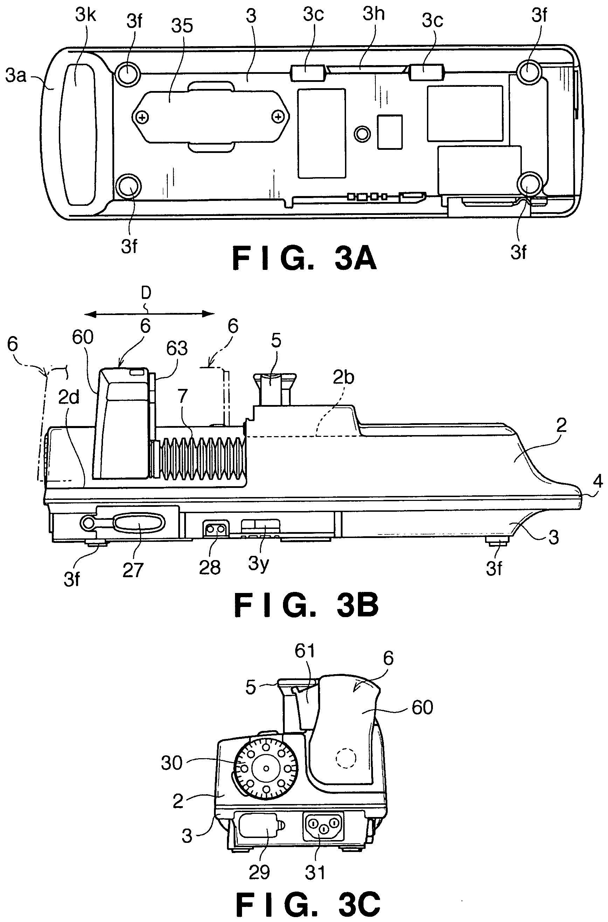

First of all, Fig. 1A is a plan view of a syringe pump 1 (to be also referred to as the device 1 hereinafter), and Fig. 1B is a front view of the device 1. Fig. 2 is a plan view of an operation panel 10 of the device 1. Fig. 3A is a plan view of the device 1. Fig. 3B is a rear view of the device 1. Fig. 3C is a right side view of the device 1.

The device 1 is also called a continuous microinfusion pump, which is used in ICU, CCU, and NICU, and has as its main object to perform nutritional support, blood infusion, infusion of a medicinal solution such as a chemotherapeutic agent or anesthetic medicinal solution. Therefore, the most part of the operation panel 10 is provided on the upper surface of the device 1 as shown in the accompanying drawings to easily display a flow rate and the like, thereby improving the operability.

The operation panel 10 is basically covered by an embossed resin sheet cover, and is provided with a drip proof design that can pass the splash proof test based on JISC0920. This provides the panel with high drip-proofness. Even if, therefore, a medicinal solution or the like drops on the cover due to, for example, carelessness, the medicinal solution can be easily wiped away and can be prohibited from entering the device 1.

Upper and lower covers 2 and 3 forming the main body of the syringe pump 1 each are integrally molded from a molding resin material, and are configured to be able to be fixed to each other at the connecting surfaces of the covers 2 and 3 with screws (not shown) through a rubber seal 4 made of, for example, silicone elastomer. This arrangement completely prohibits foreign substances such as liquids from entering the pump.

Importance is placed on accurate infusion and an improvement in operability at the time of injection. For this reason, accurate injecting operation control is implemented by microcomputer control, and an operation indicator 17 (see Figs. 1A and 1B) protruding upward at a position to provide high visibility from outside is placed at the position shown in Figs. 1A and 1B. The operation indicator 17 incorporates red and green light-emitting diodes, which turn on or blink in red or green or rotate/turn on to allow monitoring of an infusing operation state and a warning state even from a long distance, thereby reliably ensuring safety. In addition, the operation indicator 17 also incorporates a buzzer (not shown), that is, includes various types of safety-oriented warning functions.

Referring to Figs. 1A and 3A, in particular, the device 1 is compact and lightweight, and includes grip portions 2a and 3a which are integrally molded to extend from the left side surfaces of the upper and lower covers 2 and 3 through opening portions 2k and 3k. This allows the user to easily carry the device by gripping the grip portions 2a and 3a with his/her fingers inserting in the opening portions 2k and 3k. The design has been made, in particular, to facilitate the concurrent use of a plurality of devices 1, as will be described later. The grip portions give accent in the industrial design.

Referring to Fig. 3C, in particular, the right side surface of the device 1 is provided with a setting dial 30 configured to quickly set a numerical value by being rotated in accordance with its rotational speed and rotating direction. In addition, a display unit 24 placed on the left side of the operation panel 10 can display a set value in accordance with the rotation of the setting dial 30. A set numerical value such as a flow rate can be easily changed with one action using the setting dial 30. The setting dial 30 is so formed as to be easily removed for cleaning. That is, the dial 30 is configured to allow the user to easily remove it by pivoting it and moving it outside with his/her fingernail tip inserted in the gap between the dial and the upper cover.

Furthermore, the device 1 is designed to have a shape that allows multiple device use (concurrent use of many devices) (to be described later) without any unnecessary protrusions on the front and rear surfaces and the bottom surface so as to facilitate operation and allow further buildup. The specific dimensions of the device are: height H: 120 mm x width W: 360 mm x depth D: 117 mm. The weight of the device is as light as about 1.5 kg. The device uses three way power supplies including a commercial AC power supply, a built-in lithium-ion battery, and a DC 12 VA.

The charging time of the built-in battery is about 6 h (hours) and covered by the lid member 35 (see Fig. 3A) at a bottom portion of the lower cover 3 to allow easy replacement of the battery from outside. The battery is connected to a connector to be detachable. The battery has a replacement lifetime of two years or more, and is provided with constant current/constant voltage charging control to prohibit overcharge. In addition, a monitoring element is connected to the built-in battery. This element monitors the capacity of the battery, the voltage/current at the time of charging, the voltage/current at the time of discharging, and the like. This implements prohibition of overdischarge and overcharge. Using a heat-resistant lithium-ion battery, in particular, allows, if it is a new battery, the device to operate for 12 h (hours) until the generation of an alarm, and for about 12.5 h (hours) until shutdown.

The display unit 20 serves as the second display unit to display a scheduled quantity (mL), an accumulated quantity (mL), a gamma injection rate (mg/kg/h), an occlusion history, a charge history, a message indicating the contents of operation, the information of an alarm, the manufacturer name of a set syringe, and the like. This display unit and the display unit 24 to display a flow rate (mL/h) are separately provided on the operation panel 10 at a predetermined distance of about 80 to 100 mm. The display unit 20 is preferably a liquid crystal display.

To fix the syringe in a stationary state, a mount base 2b and a recess portion 2c are integrally formed with the upper cover 2 by injection molding. A syringe flange portion integrally formed with the main body of the syringe is inserted in the recess portion 2c. A clamp 5 can be manually moved in the vertical direction, and is provided to be pivotal in the arrow K direction shown in Fig. 1A. Therefore, a clamp support for supporting the clamp 5 is also integrally formed.

A slider assembly 6 to be moved and driven between the respective positions indicated by the chain lines in Fig. 3B in both the arrow D directions reciprocally moves in the space above a step portion 2d of the upper cover 2 and is coupled and fixed to a slider feeding mechanism (to be described later) through a pipe shaft and an inner clutch shaft. In the slider assembly 6, a slider cover 60 accommodates a clutch lever 61 protruding forward. Manually operating the clutch lever 61 toward the depth side allows the syringe plunger to be easily attached and detached.

The user places the syringe plunger of the syringe so as to bring it into contact with a side surface of the slider cover 60 of the slider assembly 6 after manually pressing the clutch lever 61 toward the depth side, and then releases the clutch lever 61. This makes left and right hook members 62 and 63 automatically hold the syringe plunger from the back-and-forth direction. The slider assembly 6 is fixed to the distal ends of the inner clutch shaft and pipe shaft constituting the slider feeding mechanism. The slider assembly 6 is configured to have an important function for accurately feeding a medicinal solution stored in the syringe in accordance with a set/input value or reducing the internal syringe pressure by detachably holding various different kinds of syringe plungers in a stationary state and accurately driving each syringe plunger in the infusing direction or a direction opposite to the infusing direction.

A boot 7 for waterproofing covers the pipe shaft. A lamp 25 is built in the upper surface of the slider assembly 6. The lamp 25 blinks or turns on when the left and right hooks do not properly grip the syringe plunger and a sensor (not shown) detects this state.

Referring to Figs. 1A and 2 again, the operation panel 10 includes a power switch 14, an AC/DC lamp 13, and a battery lamp 12 to turn on upon a drop in voltage, which are disposed altogether on the left distal end of the panel. A display unit 20 is disposed adjacent to these lamps. When the user fixes a syringe by using the clamp 5, the display unit 20 automatically measures the diameter of the syringe upon converting the movement amount of the clamp 5 in the vertical direction into an electrical signal, and automatically displays the volume of the fixed syringe in numerical form like 5 cc (mL), 10 cc (mL), 20 cc (mL), 30 cc (mL), 50 cc (mL), or 100 cc (mL).

In addition, a display unit 18, a remaining amount alarm lamp 19, and a clamp abnormality lamp 16 are disposed altogether almost in the middle of the device 1. The display unit 18 serves as the first display unit to sequentially display the pressures set/detected by the occlusion detection mechanism provided for the syringe pump 1 in three steps in different colors, including green, yellow, and orange. When the pressure reaches a set value (threshold), the display unit 18 displays it in red as an alarm. The clamp abnormality lamp 16 informs clamp fixation abnormality by turning on or blinking in red. The clamp abnormality lamp 16 has a shape imitating the shape of the syringe.

On the other hand, the display unit 24 incorporates preferably a 7-segment LED to display only a flow rate, and further includes an integration clear switch 15, a fast forward switch 21, a start switch 22, and a stop/mute switch 23. Note that the display unit 24 is configured to display a flow rate (mL/h) in orange when the flow rate is 0.9 mL/h or less, in green when the flow rate is 1.0 mL/h or less, and in green when the flow rate is 0.9 mL/h or less.

A sensor covered by a rubber cover (not shown) is provided near the recess portion 2c to detect that the main body of the syringe is set properly on the mount base 2b in the back-and-forth direction (X direction) in a stationary state.

Referring to Fig. 1B, a front recess portion 3h is formed in the front surface of the lower cover 3 at a position slightly shifted from the middle to the right. Abutment surfaces 3c are formed on the two sides of the front recess portion 3h. The front recess portion 3h and the abutment surfaces 3c serve to provide one lock portion to fix the device 1 to the mounting tool fixed to the infusion stand (to be described later). Four foot portions 3f are formed on the bottom surface of the lower cover 3 to fix four rubber feet.

Referring to Fig. 3B, a rear recess portion 3y is formed in the middle of the rear surface of the lower cover 3. The rear recess portion 3y serves to provide the other lock portion to fix the device 1 to the mounting tool fixed to the infusing stand (to be described later). A contact 28 is provided on the left of the rear recess portion 3y to allow the device to receive power supply after it is set on the mounting tool. An external communication connector 27 with a drip proof cover is provided adjacent to the contact 28.

Referring to Fig. 3C, a 3P commercial power supply connector 31 and a nurse call connector 29 with a waterproof cover are provided on the right side surface of the lower cover 3.

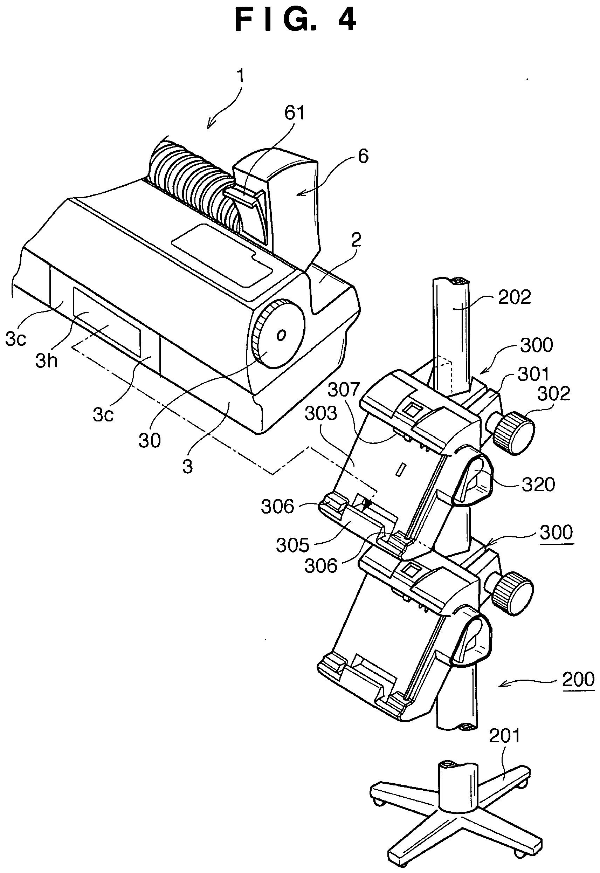

Fig. 4 is a perspective view showing an outer appearance of how the devices 1 are fixed to mounting tools 300 fixed to an infusion stand 200. The same reference numerals as in Fig. 4 denote the same components or parts which have already been described, and a description of them will be omitted. As shown in Fig. 4, the device 1 is set on the mounting tool 300 while the front recess portion 3h and the abutment surfaces 3c on the front surface of the lower cover 3 described above face forward.

Each mounting tool 300 includes a clamp device 301 to fix the device at an arbitrary height position on a pole 202 fixed to a mobile stand 201 of the infusion stand 200 so as to stand upright. The clamp device 301 is provided with a fixing knob 302. Operating the fixing knob 302 can easily detach the mounting tool 300 and attach it to poles 202 having different diameters or a bed frame.

The mounting tool 300 has an inclined mount surface 303 inclined at an angle of about 60° relative to a horizontal plane and formed as shown in Fig. 4. A protrusion 305 which is fitted in the front recess portion 3h is formed below the inclined mount surface 303. In addition, abutment surfaces 306 which come into contact with the abutment surfaces 3c described above are formed on a lower portion of the inclined mount surface 303. A pawl member 307 which is fitted in the rear recess portion 3y is pivotally provided above the inclined mount surface 303. The device can be easily attached and detached by operating the pawl member 307 between a lock position and an unlock position with a lever 320 on a side surface. Fig. 4 shows a case in which the two mounting tools 300 are provided. Obviously, however, it is possible to provide one or more mounting tools.

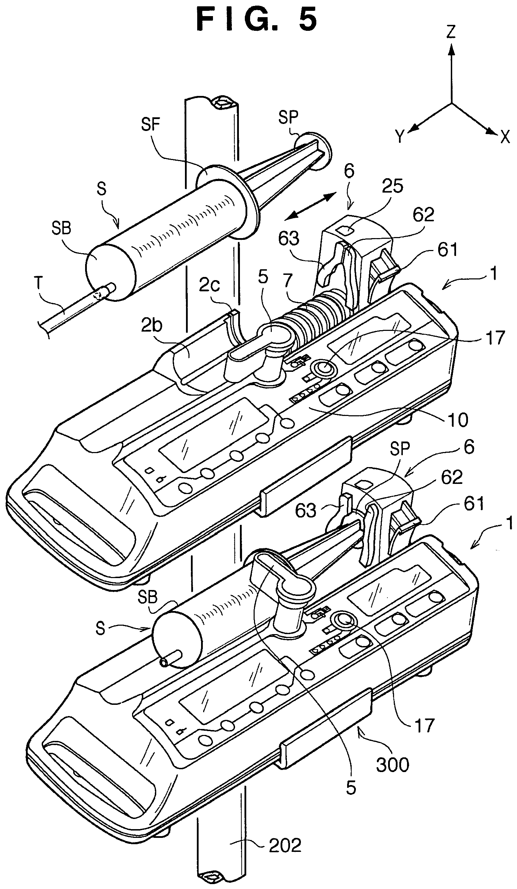

Fig. 5 is a perspective view showing an outer appearance of a state after the devices 1 are fixed to the mounting tools 300 fixed to the infusion stand 200, together with syringes S. The same reference numerals as in Fig. 5 denote the same components or parts which have already been described above, and a description of them will be omitted. As shown in Fig. 5, each device 1 is set on the corresponding mounting tool 300 while the front surfaces of the upper and lower covers 2 and 3 are positioned forward.

To set the syringe S, the user places a syringe main body SB on the mount base 2b after pivoting the clamp 5 to the front side through 90°, and sets a syringe flange SF in the recess portion 2c. The user then pulls up the clamp 5 held while being spline-fitted on the vertical projection of the clamp support (not shown) and pivots the clamp 5 to the initial position. With this operation, the syringe can be reliably fixed with tension spring force (not shown), as shown in Fig. 5. If the syringe S is not reliably fixed by the clamp 5 and/or the syringe flange SF is not reliably set in the recess portion 2c, the clamp abnormality lamp 16 turns on or blinks in red to call attention to the user.

To make the slider assembly 6 grip a syringe plunger SP of the syringe S, the user presses the clutch lever 61 in the depth direction to open the left and right hook members 62 and 63, and releases the clutch lever 61 to set the left and right hooks at the grip position. Detecting this gripped state interlockingly with a slider mechanism portion will detect that the syringe plunger SP is properly set on the slider assembly 6. For this purpose, a stopper component (not shown) is provided between the left and right hook members 62 and 63. The stopper component is biased by the effect of a torsion spring such that one end always protrudes. The stopper component retracts only when the syringe plunger SP is properly gripped. This makes it possible to detect a proper mounted state. The lamp 25 displays this state.

With the above operation, the syringe flange SF to be set in the recess portion 2c near the mount base 2b is properly set on the mount based in a stationary state in the lateral direction (Y direction). In addition, the syringe main body SB is properly set in the back-and-forth direction (X direction). A small-diameter arcuated groove portion is further integrally formed with the mount base 2b to allow a small-volume, small-diameter syringe to be held in a stationary state.

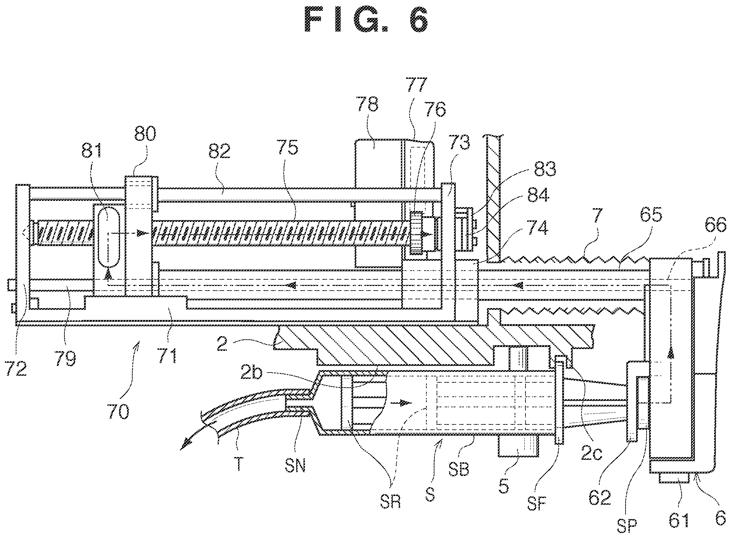

Fig. 6 is a schematic view of a slider feeding mechanism 70 built in the device 1. The same reference numerals as in Fig. 6 denote the same components or parts which have already been described above, and a description of them will be omitted. The syringe S is fixed to the upper cover 2 indicated by the hatching in Fig. 6. The syringe plunger SP is mounted on the slider assembly 6.

The slider feeding mechanism 70 includes a base 71 serving as a basal portion having excellent rigidity and vibration proofness such as an aluminum die casting base. The base 71 has integrally formed left and right wall surfaces 72 and 73 and supports, at the spatial positions shown in Fig. 6, a lead screw 75 having one end fixing a gear 76 meshed with a gear of a gear train 77, an inner clutch shaft 79, and a shaft 82. The gear train 77 obtains driving force from a stepping motor 78 fixed thereto.

The lead screw 75 is pivotally supported by a bearing (not shown). A pressure sensor 84 for detecting an occluded state in an infusing system by detecting the syringe pressure transferred as indicated by the broken line in Fig. 6 is provided on one bearing through a cantilever member 83.

A pipe shaft 65 configured to be moved in the lateral direction by a guide bush is slidably guided coaxially with the inner clutch shaft 76. A block 80 is fixed to a distal end of the pipe shaft 65. A bush made of a polyacetal resin which extends through the shaft 82 to lock it is fixed to the block 80.

A nut holder 81 is fixed to the inner clutch shaft 79. A half-nut block whose tooth portion always meshes with the tooth portion of the lead screw 75 is fixed to the nut holder 81. The meshed state is released into a free state to allow free movement only when the inner clutch shaft 79 pivots. A detection mechanism (not shown) is provided to detect that the above meshed state is released.

As described above, the slider feeding mechanism 70 is configured to transmit the rotation of the stepping motor 78, to which output shaft an encoder is fixed, as power to the final gear 76 fixed to the lead screw 75 as a feed screw through the gear train 77. This moves the nut holder 81 and pushes the syringe plunger SP, thereby infusing a medicinal solution in the syringe S. At this time, the nut holder 81 tries to move in the feeding direction, but a force acting in the opposite direction is generated by the effect of resistance generated in the syringe S and other infusion channels.

With the force generated in this manner, the overall feed screw receives a reaction force in a direction opposite to the feeding direction due to the meshed state between the half nut fixed to the nut holder 81 and the lead screw 75. As a result, the overall feed screw moves in the opposite direction, and the distal end portion of the feed screw presses the pressure sensor 84.

In addition, the force transmitted in this manner causes deflection in the pressure sensor 84. The pressure sensor 84 converts this mechanical deflection into an electrical output to cause the operation indicator 17 to display the output as an alarm informing an occlusion abnormality state and to generate (sound) a warning sound (buzzer).

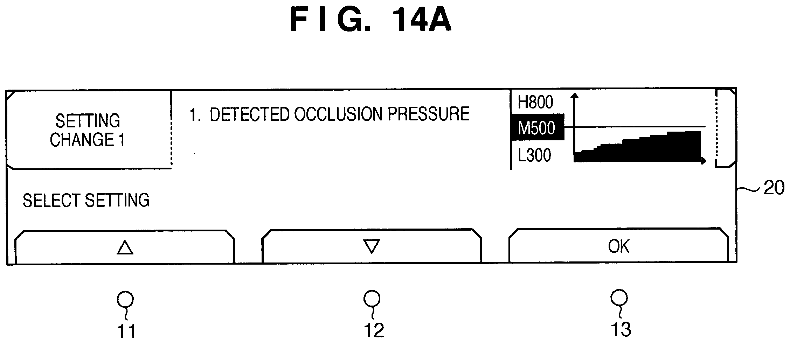

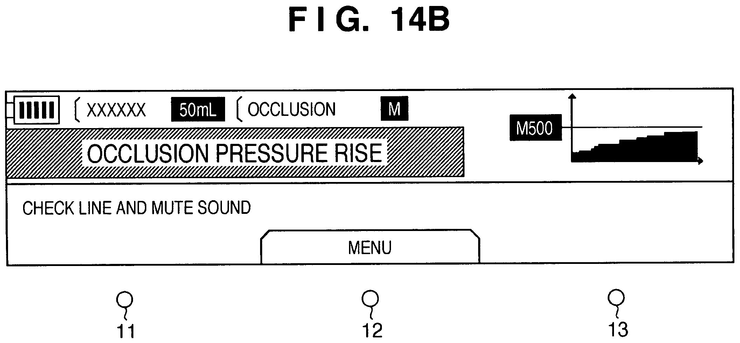

The display unit 20 of the operation panel 10 on the upper cover 2 displays the occlusion pressure (load pressure) generated by resistance in the syringe and other infusion channels. Highlighting indicates the specific value of the occlusion set pressure, that is, one of H (High): (800 ±200 mmHg), M (Medium): (500 ±100 mmHg), and L (Low): (300 ±100 mmHg) (see Figs. 14A and 14B).

The occlusion detection mechanism is formed in consideration of the sliding resistance of a syringe and configured to detect each manufacturer and a syringe volume (100 cc, 50 cc, 30 cc, 20 cc, 10 cc, or 5 cc) and perform automatic correction in accordance with the sliding resistance of the syringe. An exchangeable memory in the CPU stores the sliding resistance data of syringes available from 40 manufactures in advance and allows the user to set an arbitrary manufacturer.

On the other hand, the remaining amount detection mechanism serves as an important function when the remaining amount of medicinal solution becomes small during operation. Assume that when the device 1 continues its operation, the block 80 and the nut holder 81 move, and the syringe plunger SP moves to an arbitrary position. In this case, a metal fitting (not shown) fixed to the block fixed to the pipe shaft 65 comes into contact with the lever of a potentiometer fixed to the interior of the upper cover 2 of the main body. When the syringe plunger SP further continues to move in this contact state, the value of the potentiometer reaches a predetermined value stored in advance. At this time, this mechanism causes the operation indicator 17 to display in red as an alarm informing an abnormal state, and generates a warning sound, thereby displaying a remaining amount alarm.

When the device 1 completes the operation of infusing the drum in the above manner, the slider assembly 6 moves in a direction opposite to the feeding direction and returns to the initial position. The lever of the potentiometer then returns to the initial position due to the force of the tension spring connected to the lever. In addition, a clutch disengagement detection mechanism generates a warning sound and makes the operation indicator 17 inform abnormality when the meshed state between a half nut and the lead screw 75 is released due to accidental gripping of the clutch lever 61 or the effect of some load.

To perform abnormality detection in this manner, when the user grips the clutch lever 61 of the slider assembly 6, the half nut rotates in the opposite direction through the pipe shaft 65, and the meshed state between the thread of the lead screw 75 and the thread of the half nut is released. In addition, when the half nut rotates in this manner, a clutch sensor plate biased by a biasing means (not shown) to make its sliding surface be always in contact with the sliding surface of the half nut simultaneously rotates. As a consequence, a photosensor (transmission type) detects a lever portion on one end of the clutch sensor plate. This sensor normally blocks light. When the clutch sensor plate rotates, the sensor transmits light. Therefore, detecting this will detect that the syringe plunger SP is not gripped by the hook member. In addition, an alarm is displayed.

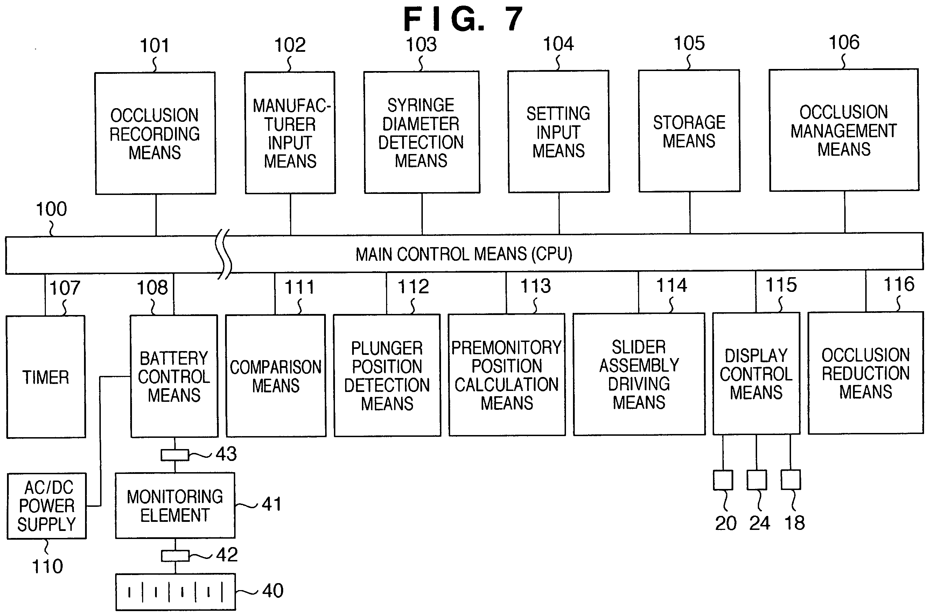

Fig. 7 is a block diagram of the syringe pump type infusion device 1. The same reference numerals as in Fig. 7 denote the same components or parts which have already been described, and a description of them will be omitted. A CPU 100 as a main control means includes an occlusion history recording means 101 to record an occlusion history, a manufacturer input means 102 to input a syringe manufacturer, a syringe diameter detection means 103 to determine the syringe diameter detected by the clamp 5, a setting input means 104 to make various settings associated with infusion, a storage means 105 to store each information, an occlusion management means 106, a battery control means 108 to manage charging and discharging of a rechargeable battery 40, a comparison means 111, a plunger position detection means 112 to detect the driving position of the syringe plunger SP, an occlusion pressure level changing means 105, a premonitory position calculation means 113, a driving means 114 to drive/control the slider assemble, a display control means 115 connected to the display units 20, 24, and 18, and an occlusion reduction means 116.

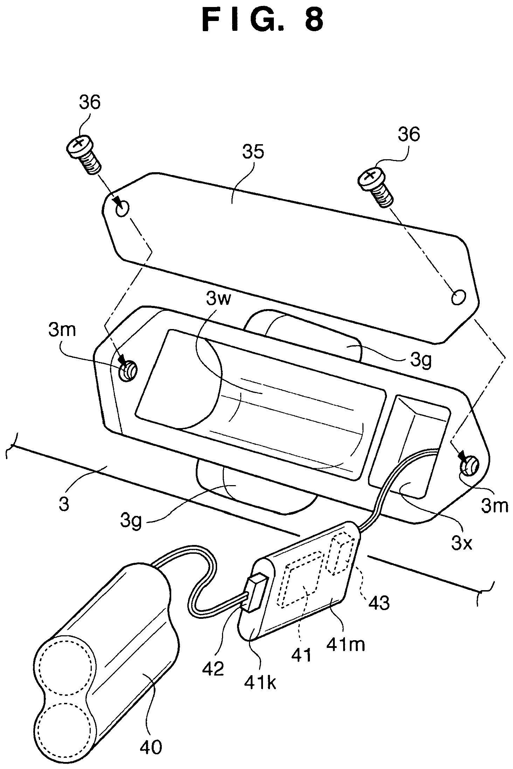

Fig. 8 is a perspective view showing an outer appearance of how the rechargeable battery 40 of a lithium-ion battery as a secondary battery is stored in the battery storage portion formed in the bottom surface of the lower cover 3. Referring to Fig. 8, the rechargeable battery 40 is obtained by accommodating, in a heat-shrinkable case, two batteries, each similar in shape to a size AA battery, after connecting them in series. The rechargeable battery 40 is detachably provided for a first connector 42 mounted on a substrate 41k, on which a monitoring element 41 is mounted, and electrically connected to the monitoring element 41.

A second connector 43 electrically and mechanically connected to the battery control means 108 described above is mounted on the substrate 41k and is finally covered with a protection cover 41m. The battery storage portion has a main storage portion 3w and a sub-storage portion 3x which are formed through a partition wall. The main storage portion 3w has a volume that enables the rechargeable battery 40 to be stored vertically. The sub-storage portion 3x has a volume that enables a connector and the like including the monitoring element 41 to be stored. The lid member 35 described above is set in the space which forms a step portion on the peripheral portion of the battery storage portion described above, and then fixed to the battery storage portion by threadably engaging two screws 36 with nuts 3m. The lid member 35 can be detached from the battery storage portion by inserting ones' fingertips into recess portions 3g.

The above arrangement can set a state in which the rechargeable battery 40 whose expiration date has passed can be detached from the first connector 42 and a state in which the rechargeable battery 40 can be detached from the second connector 43, together with the monitoring element 41. This allows maintenance personnel to replace the rechargeable battery 40 with a new battery by disconnecting the rechargeable battery 40 from the connector 42, as needed. The maintenance personnel can also arbitrarily connect another rechargeable battery 40 connected to the monitoring element 41 by detaching the rechargeable battery 40 from the connector 43, together with the monitoring element 41.

Referring to Fig. 7 again, the monitoring element 41 is connected to the battery control means 108. The monitoring element 41 monitors the voltage, current, and the like of a secondary battery which vary accompanying charging and discharging. A power supply 110 is connected to the battery control means 108 to charge the rechargeable battery 40 upon a drop in battery voltage (determine the necessity of charging) and stop charging upon full charging (determine that the battery is fully charged) by inputting voltage information temporarily stored in the monitoring element 41.

Conventionally, maintenance personnel executes refresh operation of repeating charging and discharging of the rechargeable battery 40 at a predetermined period to maintain a state of performance similar to the initial battery performance.

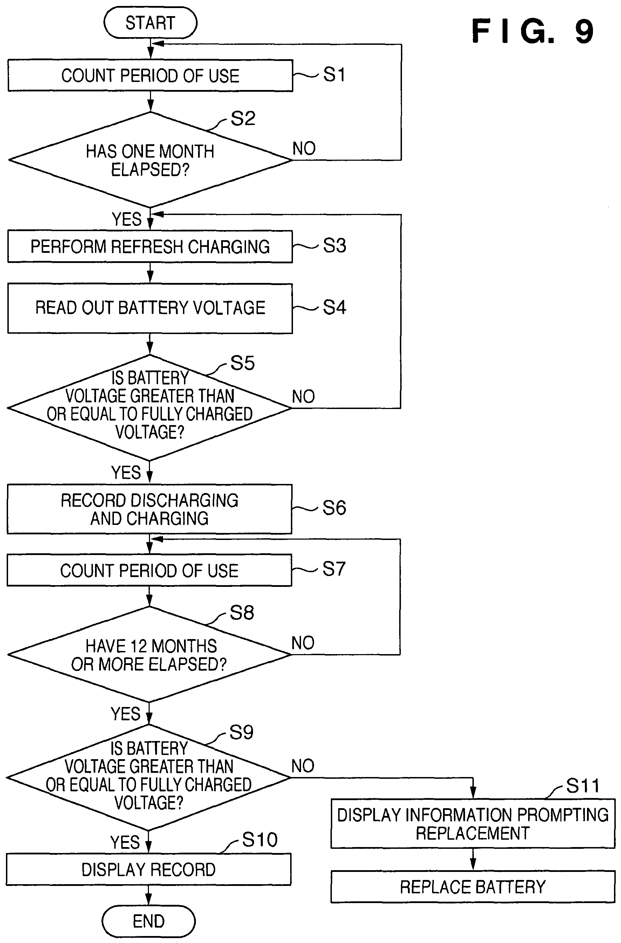

Fig. 9 is a flowchart for explaining operation including the execution/determination of refresh of the battery. Referring to Fig. 9, when the device 1 starts up, this program is executed to count the period of use of the device 1 in step S1. Subsequently, the process advances to step S2 to determine whether one month has elapsed since the start of use of the device 1. If one month has elapsed, the process advances to step S3 to execute the refresh operation of fully charging the battery after discharging. In step S4, the device reads out a battery voltage via the monitoring element 41. In step S5, the device determines based on the current and voltage whether the battery is fully charged. If the device determines in step S5 that the battery is fully charged, the process advances to step S6 to store or record the battery capacity on the secondary battery storage unit based on the battery voltage after charging. Thereafter, the process advances to step S7 to count the period of use with the lapse of time of use. If the device determines in step S8 that, for example, 12 months (one year) have elapsed, the process advances to step S9 to determine whether the battery is fully charged after the execution of refresh. If the device determines that the battery is not fully charged, the device determines the occurrence of a deterioration of the battery. In step S11, the device then displays the corresponding information regarding the display unit 20. If the device determines in step S9 that the battery is fully charged, the process advances to step S10 to record the voltage at this time. The processing is then terminated.

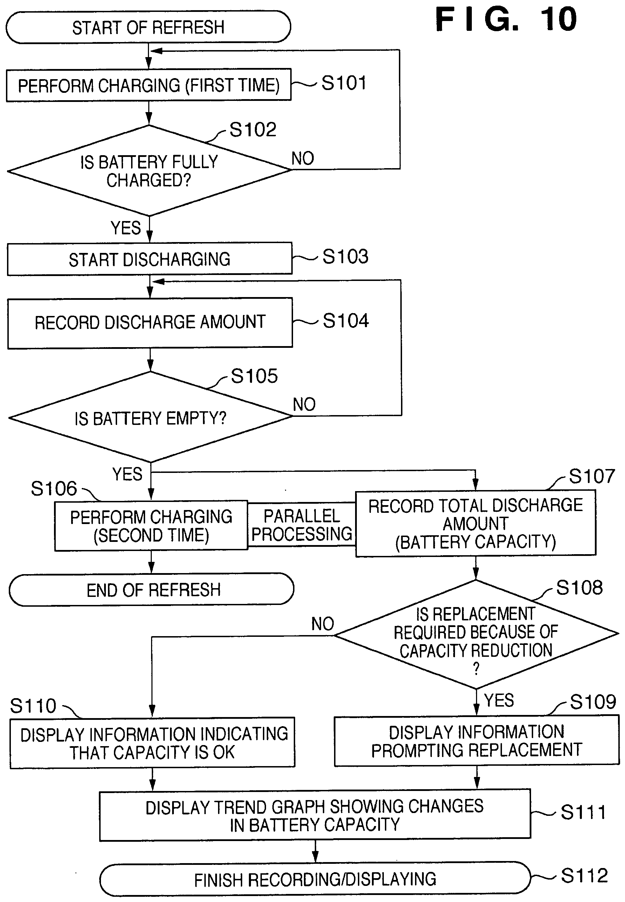

Fig. 10 is a flowchart showing a case in which a serviceman or the like manually executes refresh of the battery. Referring to Fig. 10, after the lapse of, for example, one month from the start of use, a serviceman or the like inputs refresh processing information by operating a plurality of switches including a start switch 22. In this manner, refresh starts. In step S101, the battery is charged (first time). If the device determines in step S102 that the battery is fully charged, the process advances to step S103 to start discharging. In step S104, the device records the discharge amount on the storage unit. The process then advances to step S105 to determine whether the battery is empty. If the battery is empty, the battery is charged in step S105 (second time). Concurrently with step S106, the device records the total discharge amount (battery capacity) on the storage unit in step S107. In step S108, the device determines whether it is necessary to replace the battery due to a drop in battery capacity. If it is necessary to replace the battery, the device displays information prompting replacement on the display unit 20 (step S109). If there is no need to replace the battery, the display unit 20 displays information indicating that the battery capacity is sufficient (step S110). The device then displays a trend graph showing changes in battery capacity on the display unit 20 (step S111), and stores the corresponding information in the storage unit. The device then stops displaying on the display unit 20. In this manner, the device completes the refresh processing.

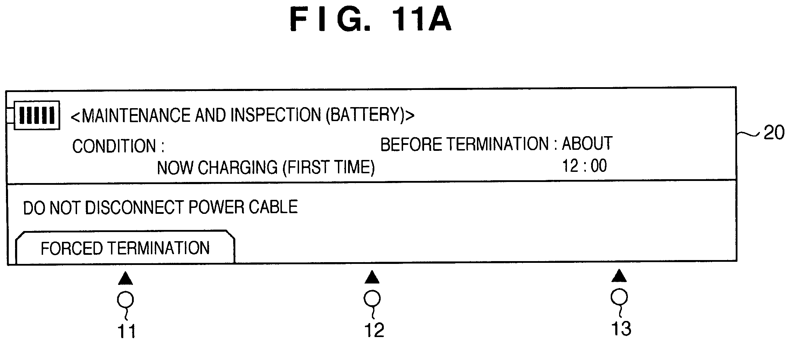

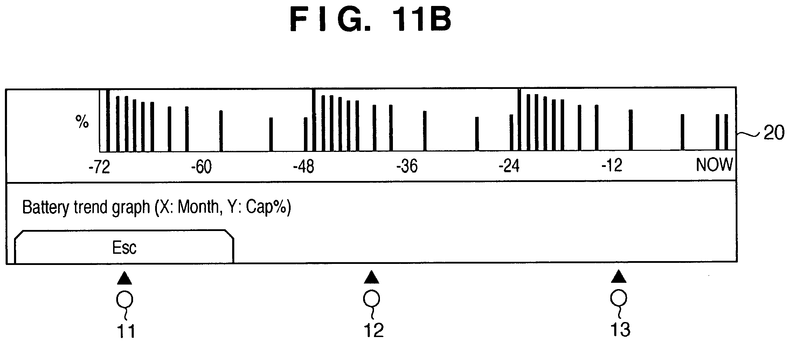

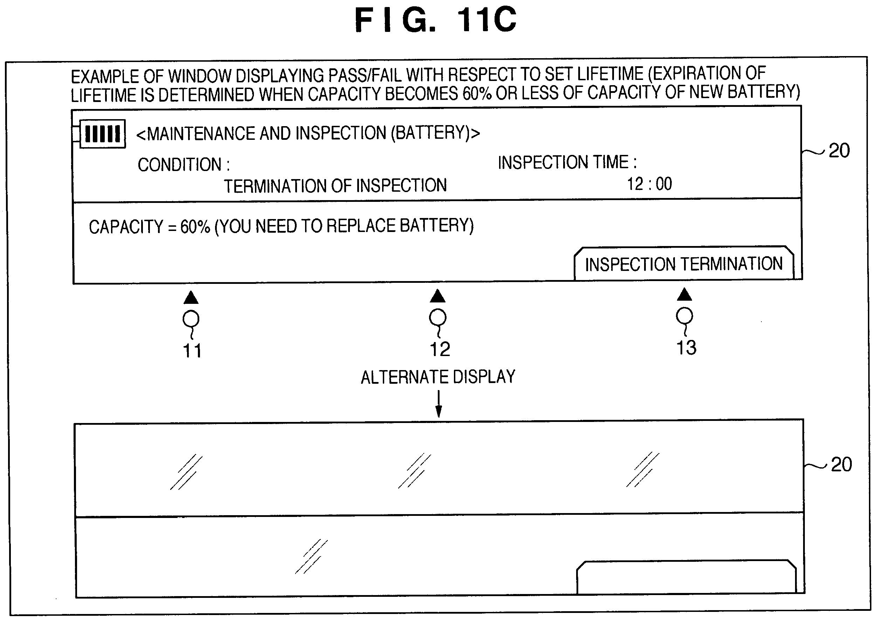

Fig. 11A shows a display example of a message on the display unit 20 at the time of the execution of refresh of the battery described above. Fig. 11B shows an example of a display window indicating the voltage trend of the battery, in %, after the execution of refresh for a period of, for example, 72 months up to the present in the form of a histogram. Fig. 11C shows an example of a window showing how a liquid crystal backlight blinks to prompt replacement because the battery cannot maintain its original performance due to a deterioration. Displaying these windows makes it possible to prompt replacement when the rechargeable battery 40 cannot have the fully charged voltage after refresh processing.

The display unit 20 includes the display portions of "forced termination", "window switching", and "inspection termination". Press keys 11, 12, and 13 are arranged at positions corresponding to the respective display portions, therefore a maintenance personnel can easily perform a battery check by pressing these press keys, as needed.

The user can then arbitrarily set (set/input) an occlusion set pressure in the above manner, and the degree of occlusion pressure is displayed. When an occlusion corresponding to each occlusion set pressure occurs, the first occlusion display unit 18 turns on or blinks in red to display a warning. With such display, however, the user cannot comprehend an occlusion trend up to an occlusion set value.

An internal syringe pressure is therefore detected during infusion by using a similar occlusion detection mechanism to detect an occluded state in the infusion line to which the syringe is connected. In addition, internal syringe pressures are stored at predetermined second intervals, for example, 5 sec intervals, from the start of infusion. When an internal syringe pressure stored in this manner becomes greater than or equal to the internal syringe pressure at the time of normal infusion, information indicating that there is a possibility that an occlusion may occur is displayed to inform, in advance, the user that there is a possibility that an occlusion may occur.

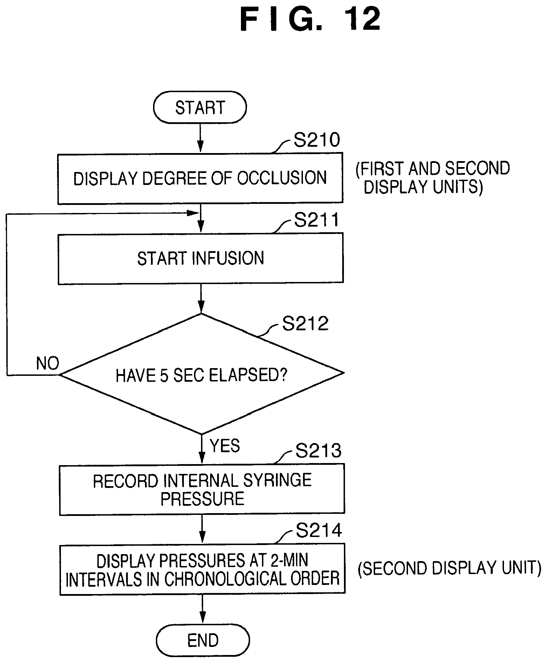

Fig. 12 is a flowchart for explaining the operation of an occlusion management means, showing an example of the operation of making the display unit 20 display internal syringe pressures, together with an occlusion degree, at least 1-min intervals in chronological order. Fig. 14A shows a display window on the display unit 20 which displays occlusions from the start time of infusion to the present, together with a set occlusion pressure. Fig. 14B shows a display example on the display unit 20 which displays a warning against the possibility of the occurrence of an occlusion.

Referring to Figs. 12 and 14A, when the user starts the device 1 and arbitrarily sets an occlusion set pressure in consideration of use conditions (the length of a tube, an adult or infant, a syringe volume, and the like), the display unit 20 displays the occlusion set pressure in highlight in step S210 (see M520 in Fig. 14A). Likewise, the display unit 18 displays the degrees of occlusion in different colors (green, yellow, and orange). Subsequently, in step S211, infusion starts. In step S212, the device determines whether predetermined seconds, for example, 5 sec, have elapsed. When 5 sec have elapsed, the process advances to step S213 to record the internal syringe pressure. After repeating steps S211, S212, and S231, the process advances to step S214 to display the occlusion pressures obtained from internal syringe pressures on the display unit 20 in chronological order at predetermined intervals, for example, 2-min intervals, for 2 h at most.

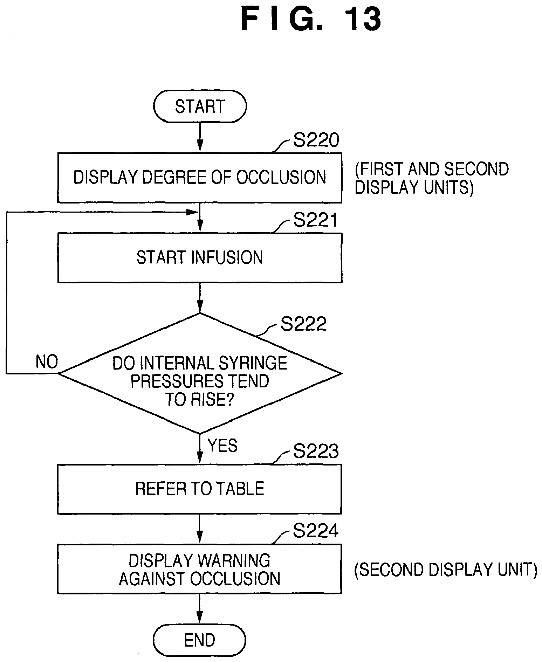

Fig. 13 is a flowchart for explaining the operation of determining the possibility of occlusion abnormality in an infusion line to which a syringe is connected, by detecting an internal syringe pressure at the time of infusion. Referring to Figs. 13 and 14B, when the user arbitrarily sets an occlusion set pressure in the above manner, the degree of occlusion corresponding to each degree of occlusion is displayed in step S220. When infusion starts in step S221, an internal syringe pressure at the time of infusion is detected by using a similar occlusion detection mechanism, and the detected internal syringe pressure is stored from the start of infusion.

In step S222, the device determines whether the internal syringe pressure tends to rise. If the device determines that the internal syringe pressure gradually rises, the process advances to step S223 to match the syringe type (the syringe volume based on the syringe diameter) and the manufacturer with the table storing occlusion set pressures. If the device determines as a result of this matching that the internal syringe pressure tends to rise, the process advances to step S224 to display character information indicating "rise in occlusion pressure" in highlight, which indicates that there is a possibility that an occlusion has occurred, and to also make the backlight blink to display a warning (see Fig. 14B).

Conventionally, the device 1 includes an internal pressure reduction mechanism to reduce the internal syringe pressure by driving the slider assembly 6 in a direction opposite to that of infusion upon detection of an occluded state. The device also includes a function of detecting the current syringe volume from the movement amount of the syringe plunger.

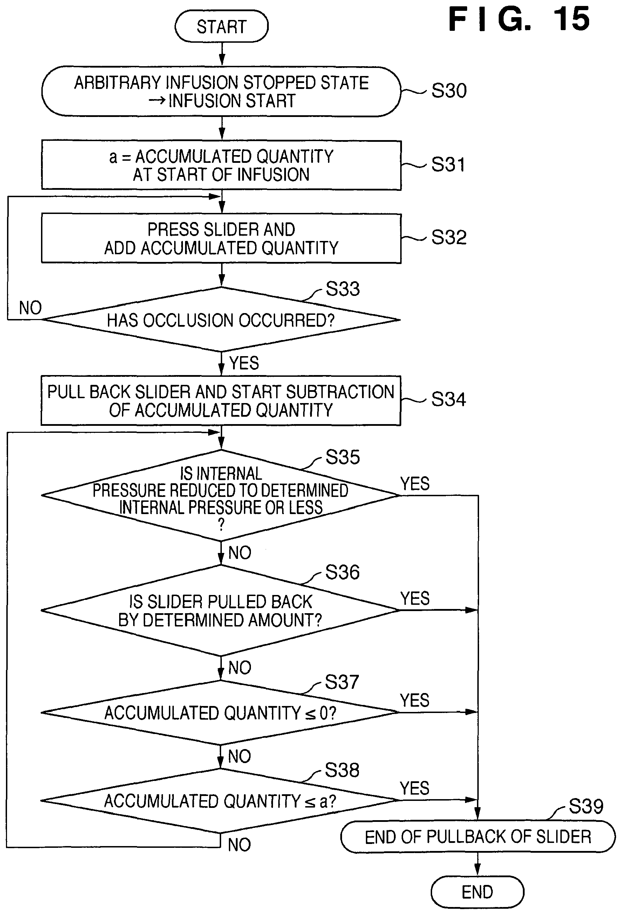

If, however, the internal pressure is reduced while the syringe volume is less than or equal to the accumulated quantity, a negative pressure is generated in the syringe. As a consequence, the medicinal solution flows backward. In the worst case, blood may be drawn from a vein. For this reason, it is possible to solve this problem by adding a condition for stopping reduction by the internal pressure reduction means when the remaining syringe volume is less than or equal to the accumulated quantity at the start of infusion. The internal pressure reduction means reduces the internal pressure in the following manner. Fig. 15 is a flowchart for explaining the operation of the occlusion reduction means 116.

Referring to Fig. 15, after the device 1 starts, the slider assembly 6 is driven to start infusion in step S30 while the internal syringe pressure is being detected. At the same time, in step S31, the device stores the accumulated quantity (position) at the start of infusion. In step S32, the device determines the occurrence/non-occurrence of an occlusion while adding an accumulated quantity corresponding to the amount by which the slider assembly 6 is driven. If the device determines in step S33 that an occlusion has occurred, the process advances to step S34 to start pullback to reduce the internal syringe pressure by driving the slider assembly 6 in a direction opposite to that of infusion. At the same time, the device starts the processing of subtracting (returning) the accumulated quantity (position) by an amount corresponding to the pullback.

Subsequently, in step S35, the device determines whether the internal syringe pressure is reduced to a specified pressure or less. If the device determines that the internal syringe pressure is reduced to the specified pressure or less, the process advances to step S39 to terminate the pullback. If the device determines in step S35 that the internal syringe pressure is not reduced to the specified pressure or less, the process advances to step S36 to determine whether pullback has been performed by a predetermined pullback amount. If the device determines that pullback has been performed by the predetermined pullback amount, the process advances to step S39 to terminate the pullback.

If the device determines in step S36 that pullback has not been performed by the predetermined pullback amount, the process advances to step S37 to determine whether the accumulated quantity is zero or less. If the accumulated quantity is zero or less, the process advances to step S39 to terminate the pullback.

If the device determines in step S37 that the accumulated quantity is not zero or less, the process advances to step S38. If the device determines that the accumulated quantity is less than or equal to the accumulated quantity stored in step S31, the process advances to step S39 to terminate the processing. If the device determines that the accumulated quantity is larger than the stored accumulated quantity, the process returns to step S35.

As described above, it is possible to prohibit suction by adding the forced termination step of stopping reduction by the internal pressure reduction means to prohibit pullback beyond the position at the start of infusion.

Obviously, the driving amount in the opposite direction is determined based on the syringe volume detected by the syringe volume detection means.

The present invention is not limited to the above embodiment and various changes and modifications can be made within the scope of the present invention. Therefore, to apprise the public of the scope of the present invention, the following claims are made.

## Claims

1. A syringe pump (1) including
infusion means for infusing a medicinal solution in a syringe (S) at a rate input by setting input means by driving a slider assembly (6) after a syringe main body (SB) filled with the medicinal solution is fixed and the syringe plunger (SP) is mounted on the slider assembly,
occlusion detection means (S33), disposed in said infusion means, for detecting an occluded state in an infusion system including the syringe by detecting an internal syringe pressure,
syringe volume detection means for detecting a syringe volume,
accumulated quantity calculation means for calculating an accumulated quantity of infusion, and
internal pressure reduction means (S34) for reducing the internal syringe pressure by driving the slider assembly in a direction opposite to an infusing direction when the occluded state is greater than or equal to a specified value, **characterized by** comprising
forced termination means (S35-S39) for stopping operation of said internal pressure reduction means when the accumulated quantity is reduced in response to operation of said internal pressure reduction means and becomes less than or equal to a specified value.

2. The syringe pump according to claim 1, **characterized in that** an amount of driving in the opposite direction is determined based on a magnitude of the syringe volume by said syringe volume detection means.

## Patentansprüche

1. Spritzenpumpe (1), umfassend:
ein Infusionsmittel zum Infundieren einer Arzneimittellösung in einer Spritze (S) mit einer Geschwindigkeit, die durch Einstellen eines Eingabemittels durch Ansteuern einer Schieberanordnung (6) eingegeben wird, nachdem ein mit der Arzneimittellösung gefüllter Spritzen-Hauptkörper (SB) fixiert ist und der Spritzenkolben (SP) auf der Schieberanordnung angebracht ist,
ein Verschlussdetektionsmittel (S33), das in dem Infusionsmittel angeordnet ist, um einen verschlossenen Zustand in einem die Spritze umfassenden Infusionssystem durch Detektieren eines Spritzeninnendrucks zu detektieren,
ein Spritzenvolumendetektionsmittel zum Detektieren eines Spritzenvolumens,
ein Ansammlungsmengenberechnungsmittel zum Berechnen einer akkumulierten Infusionsmenge, und
ein Innendruckreduzierungsmittel (S34) zum Reduzieren des Spritzeninnendrucks durch Ansteuern der Schieberanordnung in eine Richtung, die einer Infundierungsrichtung entgegengesetzt ist, wenn der verschlossene Zustand größer als oder gleich einem vorgegebenen Wert ist, **gekennzeichnet durch** Umfassen
eines Beendigungszwangsmittels (S35-S39) zum Anhalten des Betriebs des Innendruckreduzierungsmittels, wenn die akkumulierte Menge als Antwort auf den Betrieb des Innendruckreduzierungsmittels reduziert ist und weniger als oder gleich einem vorgegebenen Wert entspricht.

2. Spritzenpumpe gemäß Anspruch 1, **dadurch gekennzeichnet, dass** ein Ausmaß des Ansteuerns in die entgegengesetzte Richtung auf Basis einer Größe des Spritzenvolumens durch das Spritzenvolumendetektionsmittel bestimmt wird.

## Revendications

1. Pompe à seringue (1) comprenant
des moyens de perfusion pour perfuser une solution médicinale dans une seringue (S) à un débit d'entrée en réglant des moyens d'entrée en entraînant un ensemble coulissant (6) après qu'un corps principal de seringue (SB) rempli de la solution médicinale ait été fixé et que le piston de seringue (SP) ait été monté sur l'ensemble coulissant,
des moyens de détection d'occlusion (S33), disposés dans lesdits moyens de perfusion, pour détecter un état d'occlusion dans un système de perfusion comprenant la seringue en détectant une pression de seringue interne,
des moyens de détection de volume de seringue pour détecter un volume de seringue,
des moyens de calcul de quantité accumulée pour calculer une quantité accumulée de perfusion, et
des moyens de réduction de pression interne (S34) pour réduire la pression interne de seringue en entraînant l'ensemble coulissant dans une direction opposée à une direction de perfusion lorsque l'état d'occlusion est supérieur ou égal à une valeur spécifiée, **caractérisé en ce qu'**elle comprend
des moyens d'arrêt de force (S35-S39) pour arrêter le fonctionnement desdits moyens de réduction de pression interne lorsque la quantité accumulée est réduite en réponse au fonctionnement desdits moyens de réduction de pression interne et devient inférieure ou égale à une valeur spécifiée.

2. Pompe à seringue selon la revendication 1, **caractérisée en ce qu'**une quantité d'entraînement dans la direction opposée est déterminée sur la base d'une amplitude du volume de seringue par lesdits moyens de détection de volume de seringue.
